# EUROPÄISCHE PATENTSCHRIFT

(11) **EP 2 061 877 B1**
(45) Veröffentlichungstag und Bekanntmachung des Hinweises auf die Patenterteilung: **21.08.2013**
(21) Anmeldenummer: 07802296.9
(22) Anmeldetag: 13.09.2007
(51) Int. Cl.: C12N 5/077, C12N 5/0775

(54) **DIFFERENZIERUNG VON STAMMZELLEN**
DIFFERENTIATING OF STEM CELLS
DIFFÉRENCIATION DE CELLULES SOUCHES

(30) Priorität: 15.09.2006 DE 102006045848; 13.12.2006 DE 102006060247
(43) Veröffentlichungstag der Anmeldung: 27.05.2009
(73) Patentinhaber: Ossacur GmbH, 71720 Oberstenfeld (DE)
(72) Erfinder: BRIEST, Arne, 76227 Karlsruhe (DE); SINDET-PEDERSEN, Steen, London SW10 OJZ (GB)
(74) Vertreter: Patentanwälte Ruff, Wilhelm, Beier, Dauster & Partner
(86) Internationale Anmeldenummer: PCT/EP2007/007971
(87) Internationale Veröffentlichungsnummer: WO 2008/031588

(56) Entgegenhaltungen:
- EP-A- 1 516 926
- WO-A-2004/031369
- WO-A-2006/040615
- US-A- 6 033 906
- US-A1- 2002 146 401
- US-A1- 2004 157 326
- HEGEWALD A A ET AL: "Hyaluronic acid and autologous synovial fluid induce chondrogenic differentiation of equine mesenchymal stem cells: a preliminary study" TISSUE AND CELL, CHURCHILL LIVINGSTONE MEDICAL JOURNALS, EDINBURGH, GB, Bd. 36, Nr. 6, Dezember 2004 (2004-12), Seiten 431-438, XP004631329 ISSN: 0040-8166
- JOOS U E ET AL: "EFFECTS OF A NEW BONE-INDUCING BIOMATERIAL ON MESENCHYMAL CELLS" ARTIFICIAL ORGANS, Bd. 16, Nr. 4, 1992, Seiten 354-360, XP009093317 ISSN: 0160-564X
- WOO CHARLOTTE ET AL: "Effects of bone protein extract on human mesenchymal stem cells proliferation and differentiation" JOURNAL OF BIOMEDICAL MATERIALS RESEARCH, Bd. 79A, Nr. 3, Dezember 2006 (2006-12), Seiten 552-556, XP009093307 ISSN: 1549-3296(PRINT) & WOO CHARLOTTE ET AL: "Effects of bone protein extract on human mesenchymal stem cells proliferation and differentiation" JOURNAL OF BIOMEDICAL MATERIALS RESEARCH, [Online] Bd. 79A, Nr. 3, 20. Juni 2006 (2006-06-20), Seite 552-556, ISSN: 1552-4965 Gefunden im Internet: URL:http://www3.interscience.wiley.com/cgi -bin/fulltext/112658115/HTMLSTART> [gefunden am 2007-12-04]
- PHILIPPE COLLAS ET AL: "The A-kinase Anchoring Protein AKAP95 Is a Multivalent Protein with a Key Role in Chromatin Condensation at Mitosis", THE JOURNAL OF CELL BIOLOGY, ROCKEFELLER UNIVERSITY PRESS, US, vol. 147, no. 6, 13 December 1999 (1999-12-13), pages 1167-1179, XP007912935, ISSN: 0021-9525
- DATABASE BIOSIS [Online] BIOSCIENCES INFORMATION SERVICE, PHILADELPHIA, PA, US Februar 2001 YAN YONG-BIN ET AL: 'Two-phase unfolding pathway of ribonuclease A during denaturation induced by dithiothreitol' Database accession no. PREV200100301886
- YAN YONG-BIN ET AL: "Two-phase unfolding pathway of ribonuclease A during denaturation induced by dithiothreitol", PROTEIN SCIENCE, vol. 10, no. 2, February 2001 (2001-02), pages 321-328, XP007913024, ISSN: 0961-8368
- OIKAWA T ET AL: "A novel angiogenic inhibitor derived from Japanese shark cartilage (I). Extraction and estimation of inhibitory activities toward tumor and embryonic angiogenesis", CANCER LETTERS, NEW YORK, NY, US, vol. 51, no. 3, 15 June 1990 (1990-06-15), pages 181-186, XP026183365, ISSN: 0304-3835, DOI: DOI:10.1016/0304-3835(90)90100-C [retrieved on 1990-06-15]
- NOZOE M ET AL: "Modulation of human lymphocyte response by cartilage proteoglycans and glycosaminoglycans-A comparison between normal subjects and patients with rheumatoid arthritis", CLINICAL IMMUNOLOGY AND IMMUNOPATHOLOGY, SAN DIEGO, CA, US, vol. 12, no. 4, 1 April 1979 (1979-04-01), pages 369-381, XP026187855, ISSN: 0090-1229, DOI: DOI:10.1016/0090-1229(79)90042-4 [retrieved on 1979-04-01]

## Beschreibung

### Anwendungsgebiet und Stand der Technik

Die vorliegende Erfindung betrifft Verfahren zur mindestens teilweisen Differenzierung von Stammzellen zu mindestens einem Gewebetyp, die Verwendungen von Extrakten zur Differenzierung von Stammzellen sowie deren Verwendungen in einem Verfahren des Tissue-Engineering.

Im Rahmen von Techniken zur Gewebezüchtung, sogenanntes "Tissue engineering", betreffend eine Behandlung von Organschädigungen, insbesondere eine Verbesserung oder Wiederherstellung von biologischen Funktionen, wird der Einsatz von Stammzellen intensiv diskutiert.

Herkömmliche Differenzierungstechniken zeigen aufgrund ihrer Unausgereiftheit wesentliche Nachteile betreffend der Differenzierung sowohl von Stammzellen als auch Vorläuferzellen. Es müssen komplexe Verfahrensbedingungen zur Zelldifferenzierung eingehalten werden.

In der Praxis werden zur Gewinnung von Geweben häufig Aggregate embryonaler Stammzellen, sogenannte Embryoide, verwendet, um diese in gewünschte Gewebetypen zu differenzieren. In manchen Ländern ist aus einer Reihe von Gründen, insbesondere ethischen Erwägungen, eine massenhafte Verwendbarkeit von Embryoiden bzw. Stammzellen zur Zelldifferenzierung ausgeschlossen.

Weiterhin ist es nachteilig, dass mit aus herkömmlichen Verfahren gewonnenen adulten Stammzellen nur bedingt gewünschte Differenzierungsergebnisse erzielt werden können. In der Veröffentlichung "Effects of bone protein extract on human mesenchymal stem cells proliferation and differentiation" von C. Woo et al. wird eine Studie zur Differenzierung von mesenchymalen Stammzellen mit Hilfe eines bovinen Knochenextrakts der Anmelderin beschrieben. Hierbei ergab sich eine gewisse Wirkung des Extrakts auf die Proliferations- und Differenzierungsaktivität der Stammzellen. Insbesondere zur Behandlung schwerwiegender Erkrankungen, wie Herzinfarkt, Blutkrebs, amyotropher lateraler Sklerose (ALS), Alzheimer oder Multipler Sklerose wären jedoch weitergehende Erfolge sehr wünschenswert.

Daneben stehen Tierversuche, beispielsweise zur Kosmetik-, Hygiene- oder Medikamentenforschung, seit jeher in der Kritik. Forderungen nach einem Ausweichen auf Experimente in Zellkultur lassen sich momentan aber nur schwer erfüllen. Somit wird eine verstärkte Forschung im Bereich der Gewebezüchtung betrieben um Alternativen in Form von Gewebekulturen anbieten zu können, mit denen sich die Experimente in Petri-Schalten durchführen lassen. Es besteht daher ein vermehrter Bedarf an Techniken zur Herstellung bestimmter Gewebetypen und damit einhergehend nach Techniken zur Stammzell-Kultivierung und Differenzierung.

### Aufgabe und Lösung

Es ist daher Aufgabe der vorliegenden Erfindung, verbesserte Verfahren zur Proliferation sowie zur Differenzierung von Stammzellen in Zellkultur bereitzustellen, insbesondere zur Gewebezüchtung.

Diese Aufgabe wird gelöst durch ein Verfahren mit den Merkmalen des Anspruchs 1, durch die Verwendung von Extrakten gemäß Anspruch 4 sowie durch die Verwendung von mesenchymalen und/oder hämatopoetischen Stammzellen in einem Verfahren des Tissue-Engineering gemäß Anspruch 8. Bevorzugte Ausführungsformen des Verfahrens und der beiden Verwendungen finden sich in den abhängigen Ansprüchen 2 und 3, 5 bis 7 sowie 9 bis 11.

Das Verfahren zur mindestens teilweisen Differenzierung von mesenchymalen und/oder hämatopoetischen Stammzellen zu mindestens einem Gewebetyp umfasst eine Behandlung der Stammzellen mit Extrakten, welche Wirkstoffe und/oder Komponenten zur Differenzierung von Stammzellen aufweisen, und eine Kultivierung und Differenzierung der Stammzellen.

Unter "Stammzellen" sollen im Rahmen der Erfindung undifferenzierte Zellen oder im Wesentlichen undifferenzierte Zellen derselben Zelllinie verstanden werden. Solche Stammzellen sind in der Lage sich zu differenzieren und auf diese Weise für einen Organismus verlorene Zellen, insbesondere gestorbene oder mutierte Zellen, ersetzen zu können. Die Stammzellen sind in der Lage kontinuierlich unveränderte Tochterzellen zu produzieren, welche eine latente Fähigkeit zur Differenzierung aufweisen. Damit zeigen die Stammzellen eine charakteristische Fähigkeit zur Selbsterneuerung (self-renewal). Daneben haben die Stammzellen auch die Fähigkeit, Tochterzellen zu produzieren, welche stärkere oder unterschiedliche Differenzierungen zeigen. Mit anderen Worten, zeigen Stammzellen die Fähigkeit, Zellzyklen zu durchlaufen, und auf diese Weise wiederholt mindestens eine Tochterzelle zu erzeugen, die vergleichbar zu ihrer Mutterzelle ausgestattet ist, und sich genau wie ihre Mutterzelle differenzieren kann.

Gemäß der Erfindung werden mesenchymale und/oder hämatopoetische Stammzellen verwendet.

Um eine Klassifizierung der Stammzellen vornehmen zu können, werden diese gewöhnlich nach ihren Fähigkeiten zur Differenzierung unterteilt. In der hier vorliegenden Erfindung werden unter "totipotenten Stammzellen" solche Stammzellen verstanden, welche in der Lage sind, vollständige Organismen zu bilden. Unter "pluripotenten Stammzellen" werden solche Stammzellen verstanden, welche in der Lage sind, jede Zelllinie des Organismus zu bilden, einschließlich Keimzellen. Als Beispiel hierfür sind embryonale Stammzellen zu nennen. Unter "multipotenten Stammzellen" werden solche Stammzellen verstanden, welche in der Lage sind, mehrere Zelllinien zu bilden, aus welchen vollständige Gewebe gebildet sind. Als Beispiel hierfür sind hämatopoetische Stammzellen zu nennen. Unter "oligopotenten Stammzellen" werden solche Stammzellen verstanden, welche in der Lage sind, zumindest zwei Zelllinien von einem Gewebe zu bilden. Als Beispiel hierfür sind neuronale Stammzellen zu nennen, welche Unterklassen von Neuronen im Gehirn bilden können. Unter "unipotenten Stammzellen" werden solche Stammzellen verstanden, welche in der Lage sind, eine einzelne Zelllinie zu bilden. Als Beispiel hierfür sind spermatogonale Stammzellen zu nennen.

Unter dem Begriff "Gewebe" soll im Rahmen der hier vorliegenden Erfindung jedes aus Zellen gebildete Gewebe umfasst sein, insbesondere umfasst der Begriff "Gewebe" auch Organe. Unter dem Begriff "Organe" sollen im Rahmen der Erfindung Körperteile von Lebewesen verstanden werden, insbesondere von Wirbeltieren, vorzugsweise humanen Ursprungs. Unter Organen sollen auch aus verschiedenen Geweben zusammengesetzte Funktionseinheiten verstanden werden. Als Beispiele für Gewebe sind Knochen, Knorpel, Muskel, insbesondere Herz- oder Skelettmuskel. Als weitere Gewebe sind insbesondere Meniskus, Sehne, Band, Lunge, Blut, Haut, Leber, Niere, Drüsengewebe, Aorta, Pankreas, Hirn und/oder Nervengewebe zu nennen.

In einer Ausführungsform des erfindungsgemäßen Verfahrens weisen die Stammzellen nach der Behandlung mit den Extrakten auf ihrer Zelloberfläche spezifische Moleküle, insbesondere spezifische Moleküle für Zellen bestimmter Gewebe, und/oder eine für bestimmte Gewebe charakteristische Morphologie auf.

Vorteilhafterweise bilden die mit den Extrakten behandelten Stammzellen auf ihrer Zelloberfläche spezifische Moleküle aus, insbesondere für bestimmte Gewebetypen spezifische Moleküle. Die spezifischen Moleküle können mit Hilfe von Standardverfahren detektiert werden. Beispielsweise ist eine Detektion durch Antikörper, insbesondere markierte Antikörper, vorzugsweise fluoreszenzmarkierte Antikörper, möglich. Bei den spezifischen Molekülen handelt es sich vorzugsweise um unterschiedliche Membranoberflächenproteine, insbesondere handelt es sich um CD105 (Endoglin), CD133 (hProminin), CD271 (LNGFR) und CD45, vorzugsweise um STRO-1. Ein Vorhandensein oder Fehlen solcher Membranoberflächenproteine gewährt die Möglichkeit, unterschiedliche Zellpopulationen zu separieren sowie eine Einschätzung vorzunehmen, ob die Differenzierung in die gewünschten Gewebe stattgefunden hat.

Gemäß dem Verfahren werden zur Behandlung Extrakte eingesetzt, welche aus Zellen und/oder Gewebe aus Knorpel, Knochenmark oder Pankreas stammen und bevorzugt menschlichen und/oder tierischen Ursprungs sind.

Der Begriff "Tier" bzw. "tierischen Ursprungs" umfasst im Rahmen dieser Erfindung insbesondere sämtliche Wirbeltiere, vorzugsweise Säuger.

Verschiedene Experimente zur symmetrischen/asymmetrischen Zellteilung von Stammzellen, belegen, dass Stammzellen bzw. Vorläuferzellen in ihrem Verhalten zur Selbsterneuerung oder Differenzierung entscheidend durch ihre Umgebung beeinflusst werden. Abhängigkeiten von Proliferation bzw. der Differenzierung von Zellen ist beispielsweise von Wundverheilungen bekannt. Ein solches Verhalten von Zellen wird als Nischen-Verhalten bezeichnet.

Unter dem Begriff "Nische" sollen in der hier vorliegenden Erfindung insbesondere benachbarte Zellen, extrazelluläre Matrix bzw. deren Inhaltsstoffe sowie weitere in natürlicher Umgebung vorkommende Parameter verstanden werden, welche das Verhalten von Zellen beeinflussen, insbesondere das Verhalten von Stammzellen.

Überraschenderweise wurde festgestellt, dass Stammzellen, welche mit den Extrakten behandelt werden, eine Morphologie ausbilden, welche für die Gewebe charakteristisch ist, aus welchen die Extrakte gewonnen wurden.

Die Extrakte können insbesondere die von der Anmelderin hergestellten und kommerziell vertriebenen Extrakte COLLOSS^{®} bzw. COLLOSS^{®}E darstellen, welche Kollagen bovinen bzw. equinen Ursprungs und Wirkstoffkomplexe aufweisen.

Derartige Extrakte, welche insbesondere natürlichen Ursprungs sind, bilden vorteilhaft die Nische für die Stammzellen nach. Überraschenderweise wurde festgestellt, dass neben der Bildung spezifischer Moleküle auf der Zelloberfläche und/oder der Bildung einer charakteristischen Gewebe-Morphologie von den differenzierten Stammzellen charakteristische Substanzen der Gewebe produziert werden, aus welchen die Extrakte gewonnen wurden. Die Differenzierung wird durch die Bildung solcher Substanzen belegt. Insbesondere zeigt die Produktion sogenannter Differenzierungsmarker, dass eine Differenzierung stattgefunden hat. In Frage kommen dafür Substanzen, wie Kollagen, vorzugsweise Kollagen vom Typ I, Osteopontin, Kalzium, insbesondere eine Einlagerung von Kalzium in die Zellen, sowie eine Bildung von Aggrecan. Erfindungsgemäß können auch weitere Untersuchungsschritte an den differenzierten Stammzellen vorgesehen sein. Es ist erfindungsgemäß auch vorgesehen, die auf die erfindungsgemäße Weise hergestellten differenzierten Stammzellen oder die undifferenzierten Stammzellen auf charakteristische Transkriptionsfaktoren zu untersuchen. Hierfür eignen sich als Transkriptionsfaktoren insbesondere Octamer-4 (OCT-4), Nanog sowie Mitglieder der SOX-Familie, insbesondere SOX2, vorzugsweise SOX9.

Diese Maßnahme hat den Vorteil, dass weitere Quellen für die großtechnische Herstellung der Extrakte erschlossen werden können. Erfindungsgemäß ist es möglich in Zellkultur gezüchtete Gewebe für die Gewinnung der Extrakte zu verwenden. Auf diese Weise können Extrakte in großen Mengen erhalten werden, welche durchgängig verfügbar sind.

Offenbart ist auch ein Verfahren zur Herstellung von Extrakten, bei welchem Zellen und/oder Gewebe, insbesondere aus Knorpel, Knochenmark oder Pankreas, mindestens einem Denaturierungsvorgang zur Extraktion unterworfen werden, wobei die Zellen und/oder Gewebe bereitgestellt werden, welche Wirkstoffe und/oder Komponenten aufweisen, die Wachstum und/oder Differenzierung von Stammzellen zu mindestens einem Gewebetyp aktivieren oder inhibieren, insbesondere aktivieren.

Hinsichtlich der einsetzbaren Zellen und/oder Gewebe wird auf die bereits gegebene Beschreibung verwiesen.

Erfindungsgemäß ist es vorgesehen, die Zellen und/oder die Gewebe zur Herstellung der Extrakte aufbereitet zu verwenden, insbesondere gereinigt, entfettet und zerkleinert. Für die Denaturierung werden Guanidin und/oder Guanidiniumhydrochlorid als chaotrope Substanzen verwendet. Besonders geeignet ist eine 4 M Guanidiniumhydrochlorid-Lösung. Diese Denaturierungsmittel sind auch in Kombination verwendbar. Ferner ist es vorgesehen, die Denaturierungsmittel durch Renaturierungsschritte zu entfernen. Insbesondere sind hierunter dem Fachmann bekannte verschiedenste Dialyseverfahren bzw. Chromatographieverfahren zu verstehen, die zum Abtrennen von Denaturierungsmitteln geeignet sind. Die durch das beschriebene Verfahren hergestellten Extrakte haben vorteilhafterweise mindestens einen Renaturierungsschritt durchlaufen. Dies ist vorteilhaft, um zellschädigende Substanzen aus den Extrakten zu entfernen. Die Extrakte werden somit für ihre weitere Verarbeitung vorbereitet und ihre Zusammensetzung hinsichtlich ihrer Wirkung optimal hergestellt. Insbesondere ist ein Wechselspiel aus Denaturierungs- und anschließenden Renaturierungsschritten denkbar, vorzugsweise zur Anreicherung von Wirkstoffen in den hergestellten Extrakten.

Die hergestellten Extrakte können als lyophilisierte Extrakte vorliegen.

Insbesondere handelt es sich bei dem Produkt COLLOSS^{®} bzw. COLLOSS^{®}E um lyophilisierte Extrakte der hier beschriebenen Art, welche vorzugsweise nach dem beschriebenen Verfahren zur Herstellung der Extrakte herstellbar und in der erfindungsgemäßen Behandlung von Stammzellen einsetzbar sind.

Die nach dem beschriebenen Verfahren hergestellten Extrakte weisen Wirkstoffe und/oder Komponenten auf, die das Wachstum und/oder die Differenzierung von Stammzellen beeinflussen. Wie bereits erläutert, ist es für eine Kultivierung bzw. Differenzierung von Stammzellen äußerst vorteilhaft, eine Umgebung präsentieren zu können, die derjenigen vergleichbar ist, die in natürlichen funktionsfähigen Geweben angetroffen wird. Vorteilhafterweise werden Stammzellen durch solche Extrakte dazu angeregt, sich in die Gewebetypen zu differenzieren, aus welchen die Extrakte hergestellt worden sind. Hierbei ist es insbesondere wichtig, dass neben aktivierenden oder inhibierenden Wirkstoffen Komponenten bereitgestellt werden, welche für eine Simulierung der natürlichen Umgebung hilfreich sind.

Insbesondere kann es sich, wie im Falle der Produkte COLLOSS^{®} bzw. COLLOSS^{®}E der Anmelderin, um Strukturen handeln, welche je nach Gewebetyp unterschiedliche Ausprägungen aufweisen. Insbesondere handelt es sich hierbei um Kollagen, bevorzugt um ein Kollagen vom Typ I, welches neben einzelnen α-Kollagenmolekülen weitere Strukturen in Form von Überstrukturen aufweist. Vorteilhafterweise sind die nach dem beschriebenen Verfahren hergestellten Extrakte als biologisch unbedenklich einzustufen. Daher können die mit den Extrakten behandelten Zellen theoretisch sofort nach ihrer Behandlung, insbesondere unter Ausschluss weiterer Aufbereitung, direkt für ihre gewünschte Verwendung eingesetzt werden.

Zur Herstellung der Extrakte werden Zellen und/oder Gewebe aus Knochenmark, Knorpel oder Pankreas bereitgestellt, vorzugsweise bovinen, equinen und/oder menschlichen Ursprungs.

Der nach dem Verfahren hergestellte Extrakt umfasst Wirkstoffe und/oder Komponenten (die nicht aus Knochen stammen), die Wachstum und/oder Differenzierung von Stammzellen zu mindestens einem Gewebetyp aktivieren oder inhibieren, insbesondere aktivieren.

Durch die bereitgestellten Zellen und/oder Gewebe wird bestimmt, welche Wirkstoffe und/oder Komponenten die Extrakte aufweisen. Es kann erfindungsgemäß auch vorgesehen sein, dass zu den in den Extrakten bereits enthaltenen zusätzlich weitere Wirkstoffe und/oder Komponenten zugegeben werden. Dies dient insbesondere einer Feinregulierung des Wachstums und/oder der Differenzierung der Stammzellen. Vorzugsweise wird dies angewendet, um bestimmte Subtypen von Geweben gezielt herzustellen.

Bevorzugt umfassen die Wirkstoffe und/oder die Komponenten mindestens Leukotriene, Cytotactin, Tenascin, Laminin, Fibronektin, Zytokine, insbesondere osteogene Wirkstoffe, vorzugsweise BMP-I, BMP-II, IGF1, TGF-β1, FGF und PDGF, Kollagen, insbesondere vom Typ I.

Durch die Denaturierung von natürlichen bzw. nativen Komponenten der Extrakte werden deren native dreidimensionale Strukturen aufgebrochen, und eine anschließende Neustrukturierung der Komponenten kann stattfinden. Je nach gewählter Intensität eines Denaturierungsvorgangs bzw. je nach gewähltem Denaturierungsmittel sind einige Teile der Komponenten von der Denaturierung stärker betroffen als andere, gegebenenfalls sind einige Teile der Komponenten gar nicht betroffen. Es kann aber auch eine vollständige Denaturierung durchgeführt werden. Durch ein anschließendes Entfernen des Denaturierungsmittels durch Dialyse bilden sich weitere Strukturen der Komponenten aus, welche die Eigenschaften der Extrakte beeinflussen und von der nativen Struktur abweichen. Insbesondere ist es bei der vorliegenden Erfindung vorteilhaft, dass durch Denaturierung und Renaturierung die kompakte dreidimensionale Struktur der Komponenten gestört bzw. zerstört wird und eine insbesondere netzartige Struktur ausgebildet wird. Auf diese Weise wird eine Oberflächenvergrößerung der Komponenten bewirkt. Im Inneren der Komponenten verborgene Wirkstoffe gelangen an deren Oberfläche und sind besser für Zellen zugänglich. Die Oberflächenvergrößerung bewirkt insbesondere auch eine leichtere Diffusion der Wirkstoffe aus den Komponenten.

Es kann weiterhin vorgesehen sein, dass die Extrakte in ihren Eigenschaften modifiziert sind. Insbesondere ist es vorgesehen, die Extrakte mit Wirkstoffen herzustellen oder zu versehen, die eine spätere Differenzierung von Zellen aktivieren bzw. stimulieren und/oder verstärken. Die Wirkstoffe der Extrakte sind dabei vorzugsweise natürlichen Ursprungs, d. h. die Wirkstoffe liegen vorzugsweise in ihrer nativen Form vor. Vorteilhafterweise enthält der Extrakt sogenannte Rekrutierungsfaktoren, insbesondere Chemotaktika (Chemotaxine), beispielsweise Leukotriene, welche gezielt auf Stammzellen einwirken, vorzugsweise mesenchymale Stammzellen und/oder Knorpelvorläuferzellen. Vorteilhafterweise zählen zu den Wirkstoffen auch Substanzen, die als Familienmitglieder von Adhäsionsfaktoren bekannt sind, beispielsweise Cytotactin, Tenascin, Laminin und/oder Fibronektin. Als Wirkstoffe der Extrakte finden weiterhin vorzugsweise Wachstums- und/oder Maturationsfaktoren, vorzugsweise Zytokine, zur Proliferation und Differenzierung der Zellen Verwendung. Bei den Wachstumsfaktoren handelt es sich vorzugsweise um Knochenwachstumsfaktoren, insbesondere um BMP (bone morphogenetic proteine), vorzugsweise BMP-2, BMP-7 und/oder BMP-4, sowie um IGF (insuline-like growth factor), vorzugsweise um IGF1, und um TGF (transforming growth factor), vorzugsweise TGF-β1. Zusätzlich oder alternativ können FGF (fibroblast growth factor) und PDGF (platelet derived growth factor) als Wachstumsfaktoren enthalten sein.

Bei einem nach dem beschriebenen Verfahren hergestellten Extrakt können die Wirkstoffe und/oder Komponenten in Kombination, vorzugsweise in einem Komplex, vorliegen.

Unter einem Wirkstoffkomplex im Sinne der vorliegenden Erfindung soll eine Kombination von verschiedenen Wirkstoffen verstanden werden. Insbesondere eignet sich der Einsatz der Produkte COLLOSS^{®} bzw. COLLOSS^{®}E der Anmelderin als Extrakte, um die Differenzierung zu stimulieren.

Diese Wirkstoffe zeigen in vorteilhafter Weise überlappende Wirkfunktionen, so dass ein möglicher Aktivitätsverlust eines oder mehrerer Wirkstoffe durch die anderen, insbesondere in einem Komplex enthaltenen Wirkstoffe, übernommen bzw. kompensiert werden kann. Weiterhin können die Extrakte additive bzw. synergistische Effekte für die Differenzierung von Stammzellen aufweisen, insbesondere für eine Differenzierung von Stammzellen zu Knorpelzellen.

Die hier vorliegende Erfindung betrifft auch die Verwendung von Extrakten, umfassend Wirkstoffe und/oder Komponenten, die Wachstum und/oder Differenzierung von Stammzellen zu mindestens einem Gewebetyp aktivieren oder inhibieren, zur Differenzierung von mesenchymalen und/oder hämatopoetischen Stammzellen, herstellbar nach dem beschriebenen Verfahren. Die Extrakte werden auf dem Gebiet des Tissue-Engineering eingesetzt. Das Tissue-Engineering wird insbesondere in vitro durchgeführt.

Erfindungsgemäß werden Knorpelextrakte zur Differenzierung von mesenchymalen Stammzellen zu Chondrozyten eingesetzt. Erfindungsgemäß werden ferner Pankreasextrakte zur Differenzierung von mesenchymalen Stammzellen zu β-Zellen eingesetzt. Bevorzugt stammen die mesenchymalen Stammzellen vom Knochenmark.

Die Verwendung umfasst ferner die Differenzierung von hämatopoetischen Stammzellen unter Verwendung von Knochenmarkextrakten zu myeloiden und erythroiden Zellen.

Offenbart ist auch eine Verwendung von Stammzellen und/oder Vorläuferzellen für eine Behandlung mit Extrakten zur Therapie von Geweben.

Weitere Merkmale und Vorteile der Erfindung ergeben sich aus der nachfolgenden Beschreibung bevorzugter Ausführungsformen anhand von Beispielen in Verbindung mit den Unteransprüchen. Hierbei können die einzelnen Merkmale jeweils für sich allein oder zu mehreren in Kombination miteinander verwirklicht sein.

### Detaillierte Beschreibung des Ausführungsbeispiels

### Bovine Gelenk- bzw. Meniskus-Extrakte zur chondrozytischen in vitro-Differenzierung von humanen, aus Knochenmark gewonnenen, mesenchymalen Stammzellen (bone marrow derived mesenchymal stem cells, BMMSC)

Die Differenzierung wurde unter Verwendung von zwei unterschiedlich verdünnten Lösungen der Gelenk- bzw. Meniskus-Extrakte induziert, 1:25 und 1:50. Das Verfahren wurde zu verschiedenen Zeitpunkten unter Verwendung von Techniken zur "Real time-Polymerase-Kettenreaktion" (real time-PCR), Immunhistochemie (IHC), Enzymgekoppelten Immunadsorption (Enzyme Linked Immunosorbent Assay, ELISA) und chondro-spezifischen Färbung überwacht und untersucht.

### Material und Methoden

### 1. Herstellung der Extrakte

Für die Herstellung der Extrakte wurden Gewebe pulverisiert, vorzugsweise in tiefgefrorenem Zustand, beispielsweise unter Verwendung von flüssigem Stickstoff. Als Gewebe kommen Knorpelgewebe, insbesondere von Gelenk und/oder Meniskus, in Frage. Das erhaltene Gewebepulver wurde mit einem Lösungsmittel entfettet, insbesondere mit einem organischen Lösungsmittel, beispielsweise mit Aceton. Zweckmäßigerweise wurde eine Demineralisierung, insbesondere mit Säuren, vorzugsweise mit Mineralsäuren, wie Salzsäure, vorgenommen. Erfindungsgemäß kam 0,6 molare Salzsäure zum Einsatz. Daneben kann es zweckmäßig sein, eine Behandlung mit Chelatbildnern durchzuführen, insbesondere mit Ethylendiamintetraacetat (EDTA) und/oder Tris(hydroxymethyl)aminomethan (TRIS). Erfindungsgemäß wurden die Gewebe mit chaotropen Substanzen extrahiert, insbesondere mit Guanidin, vorzugsweise mit Guanidiniumhydrochlorid. Die so erhaltenen Extrakte wurden dialysiert, insbesondere gegen Puffer, um die chaotropen Substanzen zu entfernen. Durch die Extraktion der Gewebe wurden Kollagen und Wirkstoffe in einem einzigen Schritt isoliert. Anschließend wurden die Extrakte nach Standardverfahren lyophilisiert.

Vor der Lyophilisierung können die Extrakte auch weiteren Reinigungsschritten unterworfen werden. Insbesondere können zur Reinigung chromatographische Verfahren, wie Hochleistungsflüssigkeitschromatograpie (high performance liquid chromatography, HPLC), eingesetzt werden.

Daneben wurden als Extrakte auch Produkte der Anmelderin eingesetzt, insbesondere COLLOSS^{®} bzw. COLLOSS^{®}E. Die lyophilisierten Extrakte wurden vor ihrer Verwendung zweckmäßigerweise in Puffern aufgenommen, beispielsweise in Phosphatpuffer. Es wurden zur Lösungsherstellung 1 mg bis 10 mg Trockenmaterial der lyophilisierten Extrakte pro ml des Puffers verwendet. Die Verwendung geringer oder höher konzentrierter Lösungen in Experimenten ist auch möglich.

### 2. Isolierung der humanen, aus Knochenmark gewonnenen, mesenchymalen Stammzellen (BMMSC)

Die BMMSC sind primitive Zellen, welche im Knochenmark, bezogen auf kernhaltige Zellen, ungefähr im Verhältnis von 1:100.000 vorkommen. Die BMMSC wurden von Patienten, deren Einverständnis eingeholt wurde, unter Verwendung von Standardverfahren isoliert. Die Zellen wurden in 100 mm-Petrischalen plattiert und bei 37 °C, 95 % Luft, 5 % Kohlendioxid und 100 % Luftfeuchtigkeit inkubiert bzw. in Kulturen gehalten.

### 3. Zellmorphologie

An Tag 1, 3, 7 und 14 der Kulturen wurde die Morphologie der Zellen unter Verwendung von Differential-Interferenzkontrast-Mikroskopie (DIC-light microscopy) betrachtet. Änderungen in der Morphologie wurden dokumentiert.

### 4. Charakterisierung der BMMSC

Als Bestätigung, dass die Zellkulturen multipotente BMMSC-Populationen aufwiesen, wurde ein Vorhandensein von Zelloberflächenmarkern, insbesondere von STRO-1, verwendet. STRO-1 exprimierende Zellen wurden durch Immunfluoreszenzverfahren identifiziert. 1,5x104 BMMSC wurden in einer 12-Well-Platte auf sterilen Deckgläsern ausgesät. Die Kulturen wurden supplementiert mit einem Kontrollmedium, welches Dulbeccos modifiziertes Eagles-Medium (1.000 mg Glucose/L, L-Glutamin, NaHCO₃ und Pyridoxin·HCl, Sigma Aldrich D6046) mit 1 % Penicillin/Streptomycin und 10 % fötalem bovinen Serum (FBS) (Gibco) enthielt. Nach 36 Stunden wurden die Zellen mit 70 % Ethanol fixiert und dann drei Mal mit einem Phosphat-Puffer (Phosphate Buffered Saline, PBS) gewaschen (Cambrex). Die Zellen wurden dann mit monoklonalen Antikörpern gegen STRO-1 (R&D Systems, Minneapolis, MN) mit einer Konzentration von 10 µg/ml bei 37 °C für eine Stunde inkubiert. Die primären Antikörper wurden entfernt, und die Zellen dann drei Mal mit dem PBS gewaschen, anschließend wurden die Zellen für eine Stunde mit Anti-Maus Fluorescein-isothiocyanat-konjugierten Immunoglobulin M Antikörpern (Anti-Maus (FITC)-konjugierten IgM) (Molecular Probes) mit einer Konzentration von 1:1.000 für eine Stunde inkubiert. Die sekundären Antikörper wurden entfernt, und die Zellen drei Mal mit dem PBS gewaschen. Jede Vertiefung (Well) wurde für 10 Minuten mit Hoechst (1 µg/ml) inkubiert. Eine Negativkontrolle wurde mit dem sekundären Antikörper durchgeführt. Nach der Inkubation wurden die Zellen gewaschen, auf Träger gegeben und unter Verwendung eines Fluoreszenzmikroskops betrachtet.

### 5. Immunpräzipitation von transformierendem Wachstumsfaktor-β1 (Transforming Growth Factor-β1, TGF-β1)

Jeweils 100 µl des Gelenk- bzw. Meniskus-Extrakts wurden unter Verwendung von Anti-TGF-β1 monoklonalen Antikörpern (Chemicon) für mindestens 18 Stunden immunpräzipitiert. Ein 12 %iges (Natriumdodecylsulfat, Sodiumdodecylsulfate, SDS) SDS-Polyacrylamidgel wurde mit den Proteinen beladen, einer Elektrophorese unterzogen und auf eine Polyvinylidendifluorid (PVDF)-Membran transferiert (Western-Blot). Der Blot wurde dann mit Anti-TGF-β1 monoklonalen Antikörpern inkubiert. Erhaltene Banden wurden durch Chemolumineszenz sichtbar gemacht (Santa Cruz, USA).

### 6. Probenpräparation für Nachweise

Die Zellen wurden trypsinisiert und dann unter Verwendung von Trypanblau gezählt. 60x10³ bzw. 15x10³ mesenchymale Stammzellen wurden in einer 25 cm²-Zellkulturflasche bzw. in einer 12-Well-Platte ausgesät. Jedes Gefäß wurde mit einer entsprechenden Menge Standardmedium (Kontrollgruppe) oder einem Medium befüllt, welches den Gelenk- bzw. den Meniskus-Extrakt (boviner Ursprung) in einer verdünnten Lösung von 1:25 oder 1:50 enthielt. Als Positivkontrolle wurde rekombinantes Protein TGF-β1 (R&D Systems) mit einer Konzentration von 10 ng/ml zur chondrozytischen Differenzierung der BMMSC verwendet. Zellüberstände wurden gesammelt und bei -80 °C gelagert.

### 7. In vitro-Zytotoxizität und Zellproliferation

Die Extrakte wurden hinsichtlich ihrer Toxizität auf die kultivierten Zellen unter Verwendung von "CytoTox 96 Non-Radioactive Cytotoxicity" (Promega) untersucht. Dieses Nachweisverfahren bestimmt quantitativ die Lactatdehydrogenase (LDH), ein stabiles zytosolisches Enzym, das durch Zelllysis freigesetzt wird. Die freigesetzte LDH wurde in Kulturüberständen mit einem gekoppelten enzymatischen Nachweisverfahren bestimmt, unter Verwendung einer Umsetzung eines Tetrazoliumsalzes zu einem roten Formazanprodukt. Eine Absorbanz wurde bei einer Wellenlänge von 492 nm gemessen. Ergebnisse wurden 6 Stunden nach einer Behandlung mit den Extrakten erhalten.

Untersuchungen zur Zellproliferation wurden unter Verwendung von "CellTiter 96 Non-Radioactive" (Promega) durchgeführt. Es handelt sich um ein auf 3-(4,5-Dimethylthiazol-2-yl)-2,5-diphenyl-tetrazoliumbromid (MTT) basierendes Verfahren, welches die Eigenschaft von stoffwechselaktiven Zellen bestimmt, ein Tetrazoliumsalz umzusetzen. Beispielsweise ist die Umsetzung von schwach gelbem MTT durch zelluläre Dehydrogenasen zum dunkelblauen, wasserunlöslichen Formazan zur Aktivität der Dehydrogenasen und somit zur Überlebensrate der Zellen proportional. Die Absorbanz wird bei einer Wellenlänge von 595 nm gemessen. Die Zellen wurden gezählt und in einer Dichte von 10⁴ in einer 96-Well-Rundbodenplatte ausgesät. Die Zellproliferation wurde an dem Tag 1, 3, 7 und 14 bestimmt. 100 µl wurden jeweils in 3 Vertiefungen (Wells) gegeben. Zur Hintergrundbestimmung wurden Zellen in Vertiefungen ausgesät, welche nur das Medium enthielten. 15 µl Farbstofflösung wurden in jede Vertiefung gegeben. Die Zellen wurden für 4 Stunden bei 37 °C, 100 % Luftfeuchtigkeit sowie 5 % CO₂ inkubiert. Zwei 100 µl Aliquote wurden dann aus jedem Well entnommen und in eine 96 Wellplatte überführt. 100 µl einer Stopplösung wurden dann zu jedem Well zugegeben. Die Zellen wurden dann für minimal eine Stunde bei 37 °C, 100 % Luftfeuchtigkeit sowie 5 % CO₂ inkubiert. Die Absorbanz wurde bei der Wellenlänge von 595 nm mit einer Referenzwellenlänge von 655 nm gemessen.

### 8. Quantifizierung von Chondroitinsulfat

Die Zellen wurden mit dem PBS gewaschen und dann mit Alcianblau Färbemittel (1 % Alcianblau in 3 %iger Essigsäure) für 20 Minuten inkubiert. Die Zellen wurden durch dreimaliges Waschen mit 3 %iger Essigsäure und einmaligem Waschen mit Wasser entfärbt. Um das aufgenommene Färbemittel zu quantifizieren, wurden die Zellen in 1 %iger SDS-Lösung durch 30 minütiges Schütteln bei Raumtemperatur und anschließendem einstündigem Erhitzen auf 90 °C solubilisiert. Die Absorbanz wurde bei der Wellenlänge von 595 nm detektiert.

### 9. Histologische Färbung

### a) Toluidinblau Färbung

Die Zellen wurden gewässert und dann für zwei bis drei Minuten mit Toluidinblau Färbemittel inkubiert (pH 2). Drei konsekutive Waschschritte mit destilliertem Wasser wurden durchgeführt, nach welchen die Zellen mit 95 %igem und dann mit 100 %igem Ethanol dehydriert wurden (10maliges schnelles Eintauchen, um ein Verblassen des Färbemittels zu vermeiden). Es wurde zweimal nach jedem Schritt für je drei Minuten in Xylen gereinigt. Anschließend wurden die Zellen auf Trägern unter Verwendung eines Lichtmikroskops betrachtet.

### b) Hematoxylin und Eosin (H&E) Färbung

Die H&E Färbung wurde verwendet, um die Morphologie der Zellen und die Anwesenheit von Kalziumeinlagerungen in den Kulturen zu bewerten. 14 Tage nach Behandlung mit den Extrakten, wurden die Zellen mit 70 %igem Ethanol fixiert, für 40 Sekunden dem Hematoxylin ausgesetzt, für eine Sekunde mit saurer alkoholischer Lösung behandelt, für fünf Sekunden mit ammoniakhaltigem Wasser gewaschen, für zehn Sekunden mit Eosin gefärbt, mit Alkohol gewaschen und auf einen Träger gegeben. Die Zellen wurden unter Verwendung eines Lichtmikroskops betrachtet.

### 10. Knorpelspezifische Differenzierungsmarker

### a) SOX9

Gesamtzelluläre Ribonukleinsäure (ribonucleic acid, RNA) wurde unter Verwendung von "NudeoSpin RNA II" von Macherey-Nagel extrahiert. 1µg der RNA wurde unter Verwendung eines "one-step RT-PCR kit" (ABgene, UK) amplifiziert. Es wurden real time (RT)-PCR Primer verwendet, die für Kollagen-Typ I, SOX9, Aggrecan und Glycerinaldehyd-3-phosphat-Dehydrogenase (GAPDH) spezifisch sind. Eine Amplifikation des sogenannten "housekeeping-gene" GAPDH wurde verwendet um die Qualität der RNA zu verifizieren und eine gleichmäßige Beladung von Trenngelen mit den Proben sicherzustellen. Eine "real time reverse transcription-PCR" wurde unter Verwendung eines LightCycler (Roche Diagnostic) durchgeführt. Die RT-PCR Bedingungen wiesen einen RT-Schritt von 20 Minuten bei 61 °C auf, um einsträngige cDNA aus mRNA herzustellen, gefolgt von einer Inaktivierung von reverser Transkriptase und Denaturierung von 30 Sekunden bei 95 °C. 40 Amplifikationszyklen von einer Sekunde bei 95 °C, von 15 Sekunden bei 58 °C und von 13 Sekunden bei 72 °C, wurden durchgeführt. Abschließend wurde ein Abkühlungsschritt von 30 Sekunden bei 40 °C durchgeführt.

### b) Aggrecan

Die Zellen wurden in einer 12-well Platte in einer Dichte von 15x10³ ausgesät. 14 Tage nach der Behandlung mit den Extrakten wurden die Zellen mit dem PBS gewaschen und dann für mindestens 18 Stunden mit 70 %igem eiskalten Ethanol fixiert. Die Zellen wurden dann in 3 %igem Ziegenserum (normal goat serum NGS) für eine Stunde geblockt (blocking), danach wurden Maus Anti-Aggrecan Antikörper (Chemicon) mit einer Konzentration von 1 µg/ml verwendet und für mindestens 18 Stunden bei 4 °C inkubiert. Die primären Antikörper wurden dann entfernt, die Zellen wurden dreimal mit dem PBS gewaschen, und anschließend für eine Stunde mit Anti-Maus FITC-konjugierten IgM (Molecular Probes) in einem Verhältnis von 1:1000 inkubiert. Die sekundären Antikörper wurden entfernt und die Zellen dreimal mit dem PBS gewaschen. Eine Negativkontrolle wurde mit dem sekundären Antikörper durchgeführt. Nach der Inkubation wurden die Zellen gewaschen, auf Träger gegeben und unter Verwendung eines Fluoreszenzmikroskops betrachtet.

### 11. Analyse der zellulären Überstände

### a) Sezernierung von Kollagen-Typ I

Eine Kollagenbestimmung wurde durchgeführt, um zu überprüfen, ob in den BMMSC-Kulturen in der Gegenwart von Gelenk- und Meniskus-Extrakten eine Erhöhung des Kollagengehalts resultiert. In den Kulturmedien und Zelllysaten wurde am 14. Tag, unter Verwendung von "sircol collagen assay" (Biocolor) gemäß Herstellerangaben mit Kollagen-Typ I als Standard, säurelösliches Kollagen bestimmt.

### b) Sezernierung von Osteopontin

Die Osteopontin (OPN) Sezernierung wurde unter Verwendung eines "humanen OPN TiterZyme ELISA kit" (Assay designs) durchgeführt. Die Zellüberstände wurden am 14. Tag nach Behandlung gesammelt und nach Herstellerangaben untersucht.

### Ergebnisse

### 1. Morphologie der BMMSC

Es wurden abhängig von dem Kulturmedium Unterschiede im Gesamtauftritt der zellulären Morphologie festgestellt. An Tag 1 zeigten sowohl die BMMSC in dem Kontrollmedium als auch in dem Medium, welches die Extrakte aufwies, einen für aus Knochenmark gewonnenen Zellen typischen Spindel-Phänotyp. An Tag 3 hatte die Mehrzahl der Zellen in der Kontrollgruppe die spindelartige Morphologie beibehalten. An Tag 7 wiesen viele Zellen in dem Medium, welches die Extrakte aufwies, eine zentrale Ansammlung mit hypertrophen Zellen in ihrer Mitte auf. Dies deutete auf Chondrozyten hin. Die Kontrollgruppen behielten über den gesamten Zeitraum ihre Spindelform bei.

### 2. Charakterisierung der mesenchymalen Zellen

Der Nachweis von Zelloberflächenantigenen, so genannten stromalen Stammzellmarkern, insbesondere von STRO-1, war in den Kontrollzellen bei Passage 5 positiv. Dies zeigte die Anwesenheit der humanen BMMSC.

### 3. Die Zytotoxizität und die Proliferation

Die Untersuchungen der Zytotoxizität wurden durchgeführt, um die Zellverträglichkeit der Extrakte auf die Zellen zu belegen. Die Ergebnisse zeigten die gute Verträglichkeit der bovinen Gelenk- und Meniskus-Extrakte auf die BMMSC.

Der Proliferationsnachweis wurde durchgeführt, um das Wachstum der humanen mesenchymalen Stammzellen in der Gegenwart zweier unterschiedlich verdünnter Lösungen, 1:25 und 1:50, zu untersuchen. Die zelluläre Proliferation wurde an den Tagen 1, 3, 7, 14 und 21 evaluiert. In Zellen, welche mit den Gelenk- und Meniskus-Extrakten behandelt wurden, wurde eine verringerte Zellproliferation im Vergleich zu den Kontrollzellen festgestellt. Dies zeigt eine Zelldifferenzierung an. In unbehandelten Zellen stieg die Proliferation signifikant an. Im Gegensatz zu der Proliferationsrate der mit den Gelenk- und Meniskus-Extrakten behandelten Zellen, stieg die Zellproliferation bei der TGF-β1-Behandlung graduell an, wobei eine geringere Proliferationsrate als die der Kontrollgruppen beibehalten wurde. Da die vovinen Gelenk- und Meniskus-Extrakte viele der Wachstumsfaktoren und der Zytokine enthalten, zeigt dies, dass die Proliferation bzw. die Differenzierung durch andere Faktoren als TGF-β1 begründet sein können.

### 4. Anwesenheit von TGF-β1 in den Extrakten

Immunpräzipitationstechniken belegten die Anwesenheit von TGF-β1 in den bovinen Gelenk- und Meniskus-Extrakten.

### 5. Bestimmung von Glykosaminoglykanen (GAG)

Alcianblau- und Toluidinblau-Farbstoffe bilden Komplexe mit anionischen Glykokonjugaten (AG) wie Proteoglykanen (PG) und Glykosaminoglykanen (GAG). Es wurde Alcianblau verwendet, um die Menge von Chondroitinsulfat in Zelllysaten quantitativ zu bestimmen. Unsere Resultate zeigten einen Anstieg der Menge von Chondroitinsulfat in den mit den bovinen Extrakten behandelten Zellen im Vergleich zu den unbehandelten Zellen. Es wurde weiterhin festgestellt, dass die Zellen, welche mit den bovinen Gelenk-Extrakten behandelt wurden, mehr Chondroitinsulfat enthielten, als die Zellen, welche mit den bovinen Meniskus-Extrakten behandelt wurden. Die parallel durchgeführte Zugabe von 10 ng/ml TGF-β1 zu den Kulturen manifestierte den Anstieg des Chondroitinsulfats.

Daneben wurde die Toluidinblau-Färbung verwendet, um Glykosaminoglykane (GAG) in den BMMSC-Kulturen bei der Anwesenheit der Gelenk- und Meniskus-Extrakte histologisch nachzuweisen. Die Ergebnisse zeigten ein klares blaues Zytoplasma mit violett/purpurnen Körnchen, welche die Anwesenheit der GAG belegten.

### 6. Abwesenheit von Kalziumeinlagerungen

Die Zellen, welche mit 1:25 und 1:50 verdünnten Lösungen der bovinen Gelenk- und Meniskus-Extrakte behandelt wurden, wurden unter Verwendung des Lichtmikroskops analysiert. Eine drastische Veränderung der Morphologie behandelter Zellen wurde beobachtet, wobei gerundete Formen der Zellen eine Hypertrophie und somit eine chondrozytische Differenzierung der BMMSC anzeigten. Die unbehandelten Kontrollzellen behielten ihre Spindelform bei. Zusätzlich fehlten Anzeichen für Kalziumeinlagerungen. Die Kalziumeinlagerungen sind Beleg für eine osteozytische Differenzierung, welche für BMMSC gezeigt wurde, die mit Knochenmatrix-Extrakten, COLLOSS® und COLLOSS® E, behandelt wurden. Somit beweisen die Resultate, dass, durch die ursprünglich aus Gelenk und Meniskus gewonnenen Extrakte, nur eine chondrozytische Differenzierung der mesenchymalen Stammzellen induziert wurde.

### 7. Positive Expression der knorpetspezifischen Differenzierungsmarker

### a) SOX9

SOX9, ein Transkriptionsfaktor, welcher in der Chondrogenese eine Schlüsselrolle spielt, war im Vergleich zu unbehandelten Kontrollzellen an Tag 14 nach der Behandlung mit den Gelenk- und Meniskus-Extrakten signifikant erhöht. Diese Daten belegen die Chondrogenese.

### b) Aggrecan

Das Knorpelgewebe weist als eine seiner Strukturkomponenten das Aggrecan auf. Bei dem Aggrecan handelt es sich um ein großes Chondroitinsulfat-Proteoglykan. Es bindet neben Hyaluronan auch Verbindungsproteine und bildet auf diese Weise große Aggregate. Das Aggrecanmolekül ist aus einem Kernprotein und Glykosaminoglykanen, insbesondere Chondroitinsulfat, gebildet. Aggrecan war unter Verwendung von Maus Anti-Aggrecan Antikörpern an Tag 14 auf Proteinebene nachweisbar. Die Expression von Aggrecan war im Vergleich zu unbehandelten Zellen in chondrozytisch differenzierten humanen mesenchymalen Stammzellen stark ausgeprägt bzw. lokalisiert. Daneben wurde eine Färbung nur mit den sekundären Antikörpern durchgeführt, um die spezifische Bindung von Anti-Aggrecan Antikörpern sicherzustellen. Die Zählung der für Aggrecan positiven Zellen belegte, dass die mit dem bovinen Gelenk- und Meniskus-Extrakt behandelten BMMSC, und daneben die nur mit dem rekombinanten TGF-β1 behandelten BMMSC, im Vergleich zu den unbehandelten Kontrollproben zahlreicher vorhanden waren. Jedoch war die mRNA für Aggrecan auf Transkriptionsebene bei behandelten Zellen vermindert im Vergleich zu den unbehandelten Zellen, aber bei der TGF-β1-Behandlung signifikant erhöht. Dies könnte auf die späte Expression von Aggrecan zurückzuführen sein. Ferner können neben TGF-β1 noch weitere Zytokine an der Chondrogenese beteiligt sein.

### 8. Sezernierung von Osteopontin (OPN) und Kollagen-Typ I

Die aus Knochenmark gewonnenen mesenchymalen Stammzellen, welche mit den bovinen Gelenk- und Meniskus-Extrakten behandelt wurden, waren in ihren Überständen frei von sezerniertem OPN. Die Niveaus des OPN in den behandelten Zellen waren vergleichbar hoch, wie die in den Kontrollzellen. Weiterhin wurden als Vergleich zu den Gelenkund Meniskus-Extrakten COLLOSS® und COLLOSS® E in einer 1:50 verdünnten Lösung sowie BMP-2 und BMP-7 (OP1) mit einer Konzentration von 10 ng/ml verwendet. Diese Extrakte und rekombinanten Proteine sind als Osteogenese-Induktoren bekannt und sollten somit auch hier die OPN-Produktion induzieren. Auf der Protein-Ebene gab es bei den mit Gelenk- und Meniskus-Extrakten behandelten mesenchymalen Stammzellen gegenüber unbehandelten mesenchymalen Stammzellen ähnliche Niveaus an Typ-1-Kollagen. Die Unterschiede waren unsignifikant. Obwohl die Gewebe, aus welchen die Gelenk- und Meniskus-Extrakte gewonnen wurden, ursprünglich zu Knochengewebe benachbart waren, blieb die osteozytische Differenzierung der mesenchymalen Stammzellen nach der Behandlung mit den Gelenk- und Meniskus-Extrakte aus. Im Gegensatz dazu ist bekannt, dass COLLOSS® und COLLOSS® E die Differenzierung der aus Knochenmark stammenden mesenchymalen Stammzellen zu Osteozyten in vitro erfolgreich induzieren.

### Diskussion:

Die bovinen Gelenk- und Meniskus-Extrakte weisen einen Cocktail von Zytokinen und Wachstumsfaktoren auf, welche zur Superfamilie der transformierenden Wachstumsfaktoren (transforming growth factor, TGF) gehören. Daher wurden 10 ng/ml TGF-β1 als eine Positivkontrolle für die Induktion der Chondrogenese in den Kulturen verwendet. Die Ergebnisse zeigen, dass TGF-β1 nicht als einziger Regulator der Chondrogenese in Frage kommt. Die Induktion der Chondrogenese ist abhängig von synergistischen Effekten vieler anderer Wachstumsfaktoren und Zytokine, wie den Knochenwachstumsfaktoren (bone morphogenetic proteins, BMP). Die durch BMP und TGF induzierte Differenzierung der BMMSC zu Knorpel oder Knochen ist konzentrationsabhängig.

Die kultivierten BMMSC zeigten bei Passage 5 eine charakteristische langgestreckte Morphologie (Spindel Gestalt) und exprimierten den Zelloberflächenmarker STRO-1, welcher die Anwesenheit von Knochenmarkstammzellen bestätigte. Die Behandlung mit 1:25 und 1:50 verdünnten Lösungen, entweder von bovinen Gelenk- oder von bovinen Meniskus-Extrakten, induzierte charakteristische morphologische Veränderungen, welche an Tag 7 begannen und an Tag 14 maximal ausgeprägt waren. Es wurde die zentrale Ansammlung mit abgerundeten Zellen beobachtet, welche den für Chondrozyten typischen Phänotyp der Hypertrophie aufwiesen. Dies war bei den mit den Extrakten behandelten Kulturen mit der Abnahme an gesamtzellulärer Dichte verbunden, in höherem Ausmaß bei den mit den Meniskus-Extrakten behandelten Zellen.

Um zu beweisen, dass die Gelenk- und Meniskus-Extrakte eine chondrozytische Differenzierung von BMMSC induzieren, wurden die behandelten Zellen auf die Anwesenheit der chondro-spezifischen Marker, insbesondere SOX9, Aggrecan bzw. GAG untersucht. Die Extrakte induzierten einen signifikanten Anstieg an SOX9 mRNA, die Expression des chondro-spezifischen zellulären Proteins Aggrecan, und die Anwesenheit des Chondroitinsulfats in den Zelllysaten. Dies wurde durch die Toluidinblau-Färbung auf Glykosaminoglykane (GAG) ergänzt, welche in reichem Maße im zellulären Zytoplasma aufgefunden wurde. Die Abwesenheit von Osteopontin und Kollagen Typ-1 im zellulären Überstand bestätigte die fehlende Eigenschaft der bovinen Extrakte die osteozytische Differenzierung der BMMSC zu induzieren. Die Abwesenheit der Kalziumeinlagerung wurde in der H&E-Färbung nachgewiesen. COLLOSS®, COLLOSS® E, BMP-2 und BMP-7 (OP1) wurden als Positivkontrollen zur Induktion der osteozytischen Differenzierung verwendet. Zusammenfassend bewirken ursprünglich aus Gelenk und Meniskus stammende bovine Extrakte die chondrozytische Differenzierung von BMMSC, insbesondere in vitro.

### Einfluss von Knochenmarkextrakten auf die Zahl und die Subtypisierung von hämatopoetischen Stammzellkolonien

### 1. Hämatopoetischer Zellkolonie-Assay

Als hämatopoetischer Kolonie-Assay wurde ein CFC bzw. CFU-C Assay (colony-forming cell assay bzw. colony-forming unit culture assay) durchgeführt. Der CFC Assay wird zur Auszählung von hämatopoetischen Vorläuferzellen eingesetzt. Dazu werden die Vorläuferzellen in einer Mischung aus Kulturmedium, Wachstumsfaktoren sowie einer halbfesten Matrix suspendiert. Bei den Wachstumsfaktoren handelt es sich gewöhnlich um Zytokine. Die Wachstumsfaktoren fördern die lokale Ausdehnung und Differenzierung der hämatopoetischen Zellen in konkrete Zellkolonien. Als Matrix kommt beispielsweise Methylcellulose oder Agarose in Frage. Die suspendierten Vorläuferzellen proliferieren und differenzieren und bilden Kolonien von Blutzellen, welche mit Hilfe eines Inversions- und Dissektionsmikroskop ausgelesen werden können. Hierbei werden verschiedene Subtypen von CFCs unterschieden:
- CFU-Makrophagen (CFU-M)
- CFU-Granulozyten (CFU-G)
- CFU-Granulozyten/Makrophagen (CFU-GM)
- Burst forming unit-erythroid (BFU-E)
- CFU-Granulozyten, Makrophagen, erythroide Zellen (CFU-GEMM): frühe multipotente Vorläuferzellen

### 2. Isolierung von Knochenmarkstammzellen und aktivierten Stammzellen des peripheren Blutes

Die Knochenmarkstammzellen wurden 5 Patienten entnommen. Die Stammzellen des peripheren Blutes wurden durch Apherese einem gesunden Spender entnommen und während 5 Tage mit G-CSF aktiviert. Je 4 ml der auf diese Weise gewonnenen Stammzellproben wurden mit 4 ml einer Hank's Salzlösung verdünnt und gewaschen, wobei mehrmals leicht geschüttelt wurde. Anschließend wurden 5 ml einer Ficoll-Lösung mit den gewaschenen Stammzellproben überschichtet. Die Ansätze wurden jeweils während 30 min bei Raumtempratur zentrifugiert (200 g bzw. 1400 rpm). Danach wurde die mononukleare Zellschicht entfernt und mit Hank's Salzlösung gewaschen. Die Zellen wurden bei 800 rpm während 10 min zentrifugiert und in einem Kulturmedium aus IMD Methylcellulose resuspendiert. Anschließend wurden die Zellen in Duplikaten von 1 x 10⁵ Zellen pro Platte aliquotiert und in An- oder Abwesenheit von Knochenmarkextrakten inkubiert. Die Inkubation wurde bei 37 °C in einem feuchten Inkubator mit 5 % CO₂ bei minimaler Störung durchgeführt. Nach 14 bis 18 Tagen wurden die Platten nach CFUs (colony-forming units) gemäß Standardkriterien ausgezählt.

### 3. Methycellulose-basierte Kulturmedien für den humanen CFC Assay Folgende Kulturmedien kamen zum Einsatz:

- HSC001:: Methylcellulose-Stocklösung (frei von FBS (fötales bovines Serum) oder Zytokinen).
- HSC002:: humanes Medium auf der Basis von Methylcellulose, welches FBS, BSA (bovines Serumalbumin), L-Gln (L-Glutamin) und 2-Mercaptoethanol enthält. Die Komponenten wurden für den CFC Assay optimiert.
- HSC003:: humanes Medium vollständig aus Methylcellulose, welches FBS, BSA (bovines Serumalbumin), L-Gln (L-Glutamin) und 2-Mercaptoethanol, rekombinantes humanes SCF, rekombinantes humanes G-CSF, rekombinates humanes IL-3 und rekombinantes humanes Erythropoietin (Epo) enthält. Die Komponenten wurden für den CFC Assay optimiert und unterstützten das Wachstum von burst-forming und Kolonie bildenden erythroiden Zellen (BFU-E, CFU-E), myeloiden Zellen (CFU-GM, CFU-G, CFU-M) sowie von Zellkolonien gemischter Abstammung (CFU-GEMM).
- HSC004:: Medium vollständig aus Methylcellulose ohne Epo, welches FBS, BSA (bovines Serumalbumin), L-Gln (L-Glutamin) und 2-Mercaptoethanol, rekombinantes humanes SCF, rekombinantes humanes G-CSF und rekombinates humanes IL-3 enthält. Die Komponenten wurden für den CFC Assay optimiert und unterstützten das Wachstum von myeloiden Zellkolonien (CFU-GM, CFU-G und CFU-M).
- HSC005:: Humanes Medium, angereichert mit Methylcellulose, welches FBS, BSA (bovines Serumalbumin), L-Gln (L-Glutamin) und 2-Mercaptoethanol, rekombinantes humanes SCF, rekombinantes humanes G-CSF, rekombinates humanes IL-3, rekombinantes humanes Erythropoietin (Epo) und rekombinates humanes IL-6 enthält. Die Komponenten wurden für den CFC Assay optimiert und unterstützten das Wachstum einer gereinigten Population von Kolonie bildenden erythroiden Zellen (BFU-E, CFU-E), myeloiden Zellen (CFU-GM, CFU-G, CFU-M) sowie von gemischten Zellinien (CFU-GEMM).

### 4. Detaillierte Verfahrensanleitung für den CFC Assay in Methylcellulose basierten Medien

Die Gefäße mit dem Methylcellulose-Medium (HSC001, HSC002, HSC003, HSC004 oder HSC005) wurden aufgetaut. Danach wurden Knochenmarkextrakte in einer Konzentration von 0.0125 mg/ml und Stammzellen (1 x10⁵ Zellen pro Platte) in einem Volumenverhältnis in Dublikaten von 1:10 hinzugegeben. Die Zellen wurden gründlich gemischt. Anschließend wurden jeweils 1.5 ml davon in 35 mm Kulturplatten in Duplikaten dispergiert. Danach wurden zwei Platten mit den Proben und eine unbedeckte Wasserplatte in eine 100 mm Kulturplatte gelegt und bedeckt. Die Zellen wurden in An- und Abwesenheit der Knochenmarkextrakte (verschiedene Lösungen) bei 37 °C in einem feuchten Inkubator mit 5% CO₂ ohne Störung inkubiert.

Die Zellen proliferierten und differenzierten in einzelne Kolonien während 14 bis 18 Tagen der Inkubation.

Danach wurden die Kolonien nach ihrer Morphologie ausgezählt und charakterisiert. Hierzu wurde ein Inversionsmikroskop und eine 100 mm Kulturplatte, markiert mit einem Ausleseraster, verwendet. Zur Untersuchung der individuellen Zellmorphologie wurden einzelne Zellkolonien mit einer 200 µl Pipette aufgenommen. Dies wurde dadurch erreicht, dass eine saubere Pipette mit ungefähr 80 µl IMDM beladen wurde und anschließend die Kolonie aus dem halbfesten Medium mit Hilfe des Mikroskops geerntet wurde. Die Zellen wurden dann auf Glasträger unter Verwendung von Cytospin verteilt und mit der Wright-Giemsa Färbung angefärbt.

### 5. Ergebnisse

### 5.1 Morphologische Ergebnisse

Die Untersuchung der Zellmorphologie von Zellkolonien, welche im Medium HSC002 gezüchtet wurden, ließ nach einer Wright-Giemsa Anfärbung deutlich die Morphologie von Monozyten und Makrophagen erkennen.

### 5.2 Wirkungen der Knochenmarkextrakte (einschließlich COLLOSS^{®} E) auf die Zahl und Subtypen von hämatopoetischen Stammzellkolonien aus dem Knochenmark

In dem Kulturmedium HSC001 konnten keine Kolonien von hämatopoetischen Stammzellen beobachtet werden.

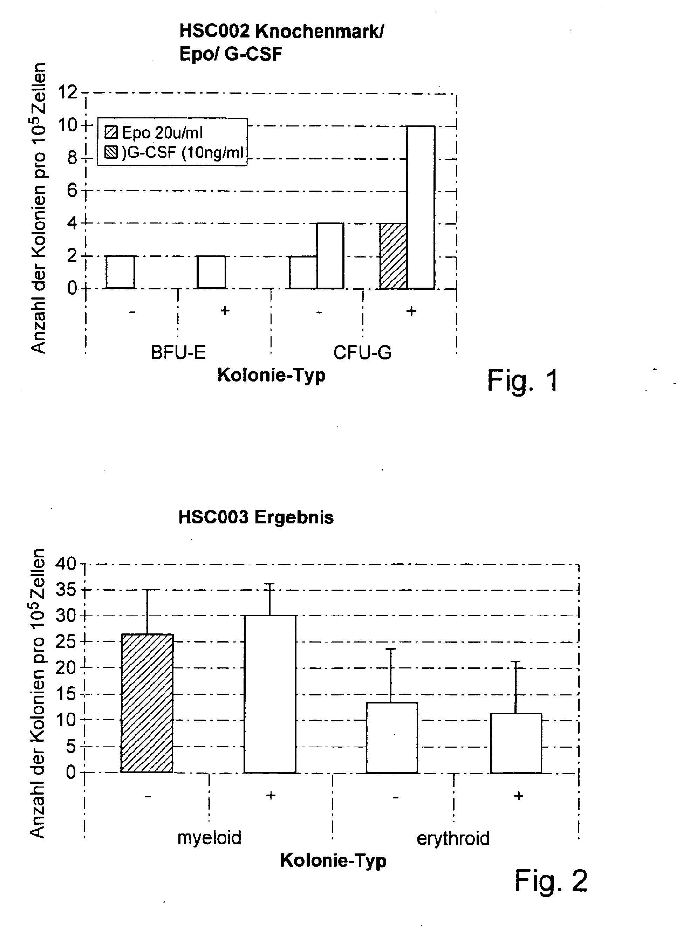

Demgegenüber führte die Zugabe von Knochenmarkextrakten zum Medium HSC002 zu einer Induktion des Wachstums von myeloiden Kolonien in Gegenwart von FBS alleine. Weiterhin führte die Zugabe von Knochenmarkextrakten zu G-CSF induzierten myeloiden Zellkolonien (CFU-G: colony-forming unit-granulozyte) zu einer signifikanten Vergrößerung sowohl der Zahl als auch der Größe der Kolonien (Figur 1: +/bedeutet in Anwesenheit von Knochenmarkextrakt/in Abwesenheit von Knochenmarkextrakt). Dagegen bewirkte die Zugabe von Knochenmarkextrakten zu HSC002 keine Erhöhung der Anzahl der Epostimulierten erythroiden Kolonien.

Weiterhin führten die hinzugegebenen Knochenmarkextrakte zum Medium HSC003 zu keinen merklichen Veränderungen im Wachstum von myeloiden oder erythroiden Zellkolonien (Figur 2: +/- bedeutet in Anwesenheit von Knochenmarkextrakt/in Abwesenheit von Knochenmarkextrakt). Dies ist wahrscheinlich auf die Anwesenheit von überlappenden Zytokinen zwischen dem Medium HSC003 und den Knochenmarkextrakten zurückzuführen. Allerdings könnte das Medium HSC003 aber auch eine Maskierung der Wirkungen der Knochenmarkextrakte bewirken. Jedoch waren die Kolonie bildenden Zellen, welche in An- oder Abwesenheit von Knochenmarkextrakten wuchsen, meistens myeloiden Ursprungs.

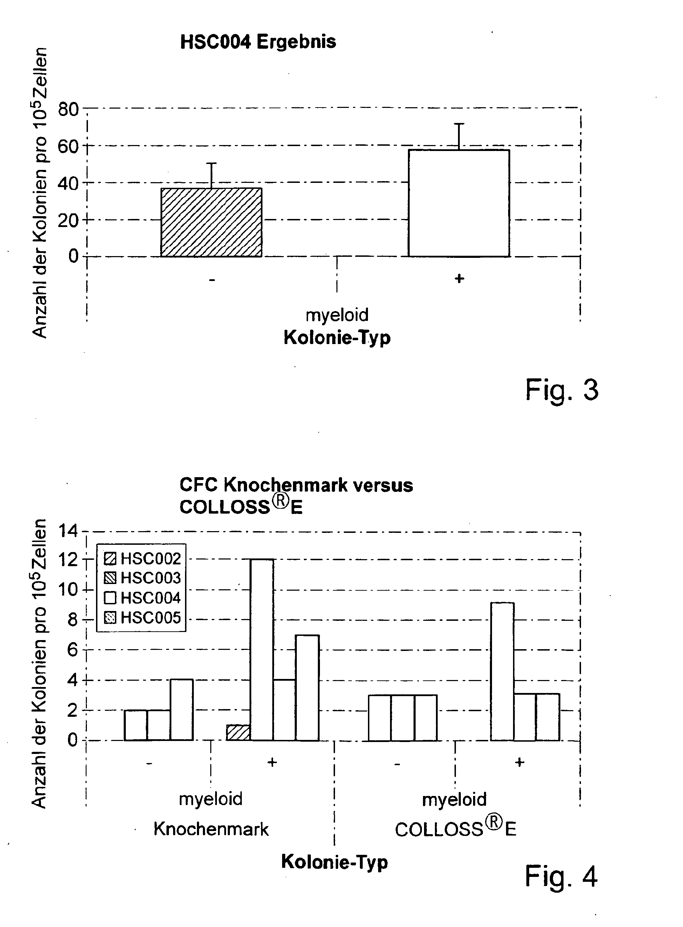

Die Zugabe von Knochenmarkextrakten zu dem Medium HSC004 bewirkte insgesamt (und bei jeder einzelnen Proben) eine deutliche Wachstumsverstärkung von Kolonie bildenden Zellen mit myeloidem Potential (Figur 3: +/- bedeutet in Anwesenheit von Knochenmarkextrakt/in Abwesenheit von Knochenmarkextrakt).

Die Zugabe von Knochenmarkextrakten zu dem Medium HSC005 führte zu keinen merklichen Veränderungen des Wachstums von Kolonie bildenden Zellen mit myeloidem oder erythroidem Potential. Auch diese Beobachtung ist wahrscheinlich auf die Anwesenheit von überlappenden Zytokinen zwischen dem Medium HSC005 und den Knochenmarkextrakten zurückzuführen. Ebenso könnte das Medium HSC005 aber auch eine Maskierung der Wirkungen der hinzugegebenen Knochenmarkextrakte bewirken. Jedoch waren die Kolonie bildenden Zellen, welche in An- oder Abwesenheit von Knochenmarkextrakten wuchsen, meistens myeloiden Ursprungs.

Weiterhin zeigten Knochenmarkextrakte im Vergleich zu dem Knochenextrakt COLLOSS^{®} E (ein Produkt der Anmelderin, das in der Beschreibung näher erläutert ist) ein höheres Wachstumspotential von Kolonie bildenden Zellen. Allerdings bewirkte COLLOSS^{®} E eine verstärkte Bildung von myeloiden Kolonien in HSC003 (Figur 4: +/- bedeutet in Anwesenheit von Knochenmarkextrakt/in Abwesenheit von Knochenmarkextrakt). Die Konzentration von COLLOSS^{®} E betrug 0.14 mg/ml und war somit zehn Mal höher als die Konzentration der verwendeten Knochenmarkextrakte (0.0125 mg/ml).

### 5.3 Wirkungen der Knochenmarkextrakte auf die Zahl und Subtypen von hämatopoetischen Stammzellkolonien, welche von aktivierten Stammzellen des peripheren Blutes abgeleitet sind.

Myeloide und erythroide Zellkolonien wurden in An- und Abwesenheit von Knochenmarkextrakten beobachtet.

Die Zugabe von Knochenmarkextrakten zum Medium HSC002 führte nicht zu einer signifikanten Erhöhung des Wachstums der myeloiden Zellkolonien.

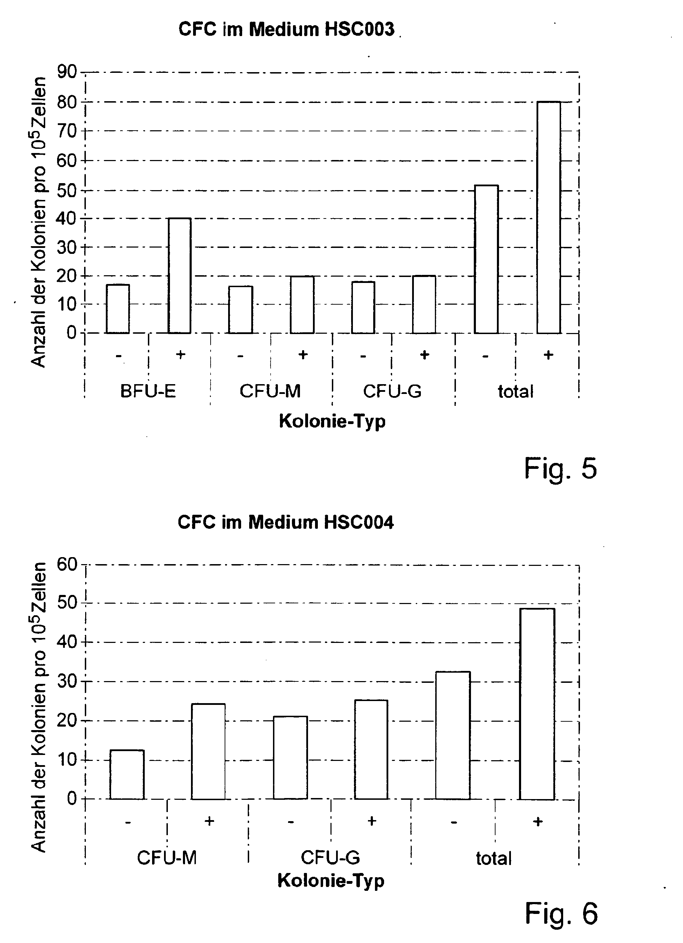

Allerdings konnte nach Zugabe von Knochenmarkextrakten zum Medium HSC003 eine signifikant erhöhte Bildung von erythroiden Zellkolonien (BFU-E: burst forming unit-erythroid) sowie insgesamt eine deutlich verstärkte Bildung von Zellkolonien in HSC003 festgestellt werden (Figur 5: +/- bedeutet in Anwesenheit von Knochenmarkextrakt/in Abwesenheit von Knochenmarkextrakt).

Weiterhin bewirkten die Knochenmarkextrakte im Medium HSC004 ein deutlich verstärktes Wachstum von Kolonie bildenden Zellen mit myeloidem Potential (Figur 6: CFU-M: colony-forming unit-Macrophage; +/- bedeutet in Anwesenheit von Knochenmarkextrakt/in Abwesenheit von Knochenmarkextrakt). Die Zugabe von Knochenmarkextrakten zu HSC004 verursachte außerdem eine deutliche Ehhöhung der Kolonie bildenden Zellen insgesamt.

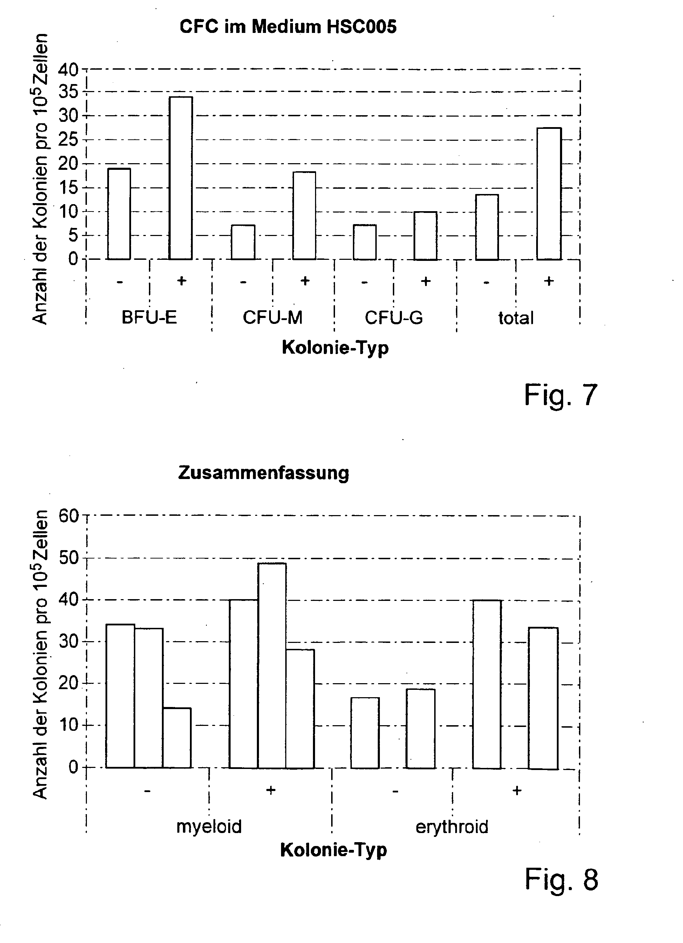

Ebenso konnte nach Zugabe der Knochenmarkextrakte zum Medium HSC005 eine signifikant verstärkte Bildung von erythroiden und myeloiden Zellkolonien festgestellt werden (Figur 7: BFU-E: burst forming unit-erythroid und CFU-M: colony-forming unit-Macrophage; +/- bedeutet in Anwesenheit von Knochenmarkextrakt/in Abwesenheit von Knochenmarkextrakt).

Zusammenfassend lässt sich daher sagen, dass ausgehend von aktivierten Stammzellen des peripheren Blutes die Bildung von Zellkolonien mit myeloider Abstammung in den Medien HSC004 und HSC005 verstärkt wird. Weiterhin lässt sich eine verstärkte Bildung von Zellkolonien mit erythroider Abstammung in den Medien HSC003 und HSC005 nach Zugabe der Knochenmarkextrakte feststellen (Figur 8: +/- bedeutet in Anwesenheit von Knochenmarkextrakt/in Abwesenheit von Knochenmarkextrakt).

### Behandlung von mesenchymalen Stammzellen aus dem Knochenmark mit einem Pankreas-Extrakt

Nach Behandlung von mesenchymalen Stammzellen aus dem Knochenmark eines Patienten mit einem Pankreas-Extrakt konnte in den behandelten Stammzellen die Expression von Glut2 nachgewiesen werden. Im Falle von Glut2 handelt es sich um ein spezifisches Transporterprotein der β-Zellen des Pankreas.

## Patentansprüche

1. Verfahren zur mindestens teilweisen Differenzierung von mesenchymalen und/oder hämatopoetischen Stammzellen zu mindestens einem Gewebetyp, umfassend:
- Behandlung der Stammzellen mit Extrakten, welche Wirkstoffe und/oder Komponenten zur Differenzierung von Stammzellen aufweisen, und
- Kultivierung und Differenzierung der Stammzellen,
**dadurch gekennzeichnet, dass** zur Herstellung der Extrakte Knorpel, Knochenmark oder Pankreas mindestens einem Denaturierungsvorgang zur Extraktion unterworfen wird, wobei
- Zellen und/oder Gewebe aus Knorpel, Knochenmark oder Pankreas bereitgestellt,
- pulverisiert, vorzugsweise in tiefgefrorenem Zustand,
- entfettet, vorzugsweise mit einem organischen Lösungsmittel,
- demineralisiert, vorzugsweise mit Säuren,
- extrahiert mit Guanidin und/oder Guanidiniumhydrochlorid als chaotrope Substanzen und
- dialysiert, vorzugsweise gegen Puffer, werden und wobei
- mesenchymale Stammzellen unter Verwendung von Knorpelextrakten zu Chondrozyten,
- mesenchymale Stammzellen unter Verwendung von Pankreasextrakten zu β-Zellen und/oder
- hämatopoetische Stammzellen unter Verwendung von Knochenmarkextrakten zu myeoloiden und erythroiden Zellen differenziert werden.

2. Verfahren gemäß Anspruch 1, **dadurch gekennzeichnet, dass** die Stammzellen nach der Behandlung mit den Extrakten auf ihrer Zelloberfläche spezifische Moleküle für Zellen bestimmter Gewebe und/oder eine für bestimmte Gewebe charakteristische Morphologie aufweisen.

3. Verfahren gemäß Anspruch 1 oder 2, **dadurch gekennzeichnet, dass** zur Behandlung Extrakte eingesetzt werden, welche menschlichen und/oder tierischen Ursprungs sind.

4. Verwendung von Extrakten, umfassend Wirkstoffe und/oder Komponenten, die Wachstum und/oder Differenzierung von Stammzellen zu mindestens einem Gewebetyp aktivieren oder inhibieren, zur Differenzierung von mesenchymalen und/oder hämatopoetischen Stammzellen, herstellbar nach einem Verfahren; bei welchem Knorpel, Knochenmark oder Pankreas mindestens einem Denaturierungsvorgang zur Extraktion unterworfen wird, wobei
- Zellen und/oder Gewebe aus Knorpel, Knochenmark oder Pankreas bereitgestellt,
- pulverisiert, vorzugsweise in tiefgefrorenem Zustand,
- entfettet, vorzugsweise mit einem organischen Lösungsmittel,
- demineralisiert, vorzugsweise mit Säuren,
- extrahiert mit Guanidin und/oder Guanidiniumhydrochlorid als chaotrope Substanzen und
- dialysiert, vorzugsweise gegen Puffer, werden und wobei
- mesenchymale Stammzellen unter Verwendung von Knorpelextrakten zu Chondrozyten,
- mesenchymale Stammzellen unter Verwendung von Pankreasextrakten zu β-Zellen und/oder
- hämatopoetische Stammzellen unter Verwendung von Knochenmarkextrakten zu myeoloiden und erythroiden Zellen differenziert werden.

5. Verwendung gemäß Anspruch 4, **dadurch gekennzeichnet, dass** der Knorpel, das Knochenmark oder die Pankreas menschlichen und/oder tierischen Ursprungs ist.

6. Verwendung gemäß Anspruch 4 oder 5, **dadurch gekennzeichnet, dass** die Wirkstoffe und/oder die Komponenten mindestens Leukotriene, Cytotactin, Tenascin, Laminin, Fibronektin, Zytokine, osteogene Wirkstoffe, insbesondere BMP-1, BMP-2, IGF1, TGF-β1, FGF und PDGF, Kollagen, insbesondere vom Typ-I, umfassen.

7. Verwendung gemäß einem der Ansprüche 4 bis 6, **dadurch gekennzeichnet, dass** die Wirkstoffe und/oder Komponenten in Kombination und/oder in einem Komplex vorliegen.

8. Verwendung von mesenchymalen und/oder hämatopoetischen Stammzellen in einem Verfahren des Tissue-Engineering, umfassend eine Behandlung der Stammzellen mit Extrakten, umfassend Wirkstoffe und/oder Komponenten, die Wachstum und/oder Differenzierung von Stammzellen zu mindestens einem Gewebetyp aktivieren oder inhibieren, wobei die Extrakte herstellbar sind nach einem Verfahren, bei welchen Knorpel, Knochenmark oder Pankreas mindestens einem Denaturierungsvorgang zur Extraktion unterworfen wird, **dadurch gekennzeichnet, dass**
- Zellen und/oder Gewebe aus Knorpel, Knochenmark oder Pankreas bereitgestellt,
- pulverisiert, vorzugsweise in tiefgefrorenem Zustand,
- entfettet, vorzugsweise mit einem organischen Lösungsmittel,
- demineralisiert, vorzugsweise mit Säuren,
- extrahiert mit Guanidin und/oder Guanidiniumhydrochlorid als chaotrope Substanzen und
- dialysiert, vorzugsweise gegen Puffer, werden und wobei
- mesenchymale Stammzellen unter Verwendung von Knorpelextrakten zu Chondrozyten,
- mesenchymale Stammzellen unter Verwendung von Pankreasextrakten zu β-Zellen und/oder
- hämatopoetische Stammzellen unter Verwendung von Knochenmarkextrakten zu myeoloiden und erythroiden Zellen differenziert werden.

9. Verwendung gemäß Anspruch 8, **dadurch gekennzeichnet, dass** der Knorpel, das Knochenmark oder die Pankreas menschlichen und/oder tierischen Ursprungs ist.

10. Verwendung gemäß Anspruch 8 oder 9, **dadurch gekennzeichnet, dass** die Wirkstoffe und/oder die Komponenten mindestens Leukotriene, Cytotactin, Tenascin, Laminin, Fibronektin, Zytokine, osteogene Wirkstoffe, insbesondere BMP-1, BMP-2, IGF1, TGF-β1, FGF und PDGF, Kollagen, insbesondere vom Typ-I, umfassen.

11. Verwendung gemäß einem der Ansprüche 8 bis 10, **dadurch gekennzeichnet, dass** die Wirkstoffe und/oder Komponenten in Kombination und/oder in einem Komplex vorliegen.

## Claims

1. A method for the at least partial differentiation of mesenchymal and/or hematopoietic stem cells to at least one tissue type, comprising:
- treatment of the stem cells with extracts which include active substances and/or components for the differentiation of stem cells, and
- cultivation and differentiation of the stem cells,
**characterized in that**
for producing the extracts cartilage, bone marrow or pancreas are subjected to at least one denaturation operation for the extraction, wherein
- cells and/or tissues from cartilage, bone marrow or pancreas are provided,
- powdered, preferably in the deep-frozen state,
- defatted, preferably using an organic solvent,
- demineralized, preferably using acids,
- extracted using guanidine and/or guanidinium hydrochloride as chaotropic substances, and
- dialyzed, preferably against buffers, and
wherein differentiation is effected of
- mesenchymal stem cells to chondrocytes using cartilage extracts,
- mesenchymal stem cells to ß-cells using pancreas extracts, and/or
- hematopoietic stem cells to myeloid and erythroid cells using bone marrow extracts.

2. The method as claimed in claim 1, **characterized in that** the stem cells exhibit, after the treatment with the extracts, on their cell surface specific molecules for cells of particular tissues, and/or a morphology characteristic of particular tissues.

3. The method as claimed in claim 1 or 2, **characterized in that** extracts which are of human and/or animal origin are employed for the treatment.

4. Use of extracts comprising active substances and/or components which activate or inhibit growth and/or differentiation of stem cells to at least one tissue type, for the differentiation of mesenchymal and/or hematopoietic stem cells, that may be prepared according to a method, wherein cartilage, bone marrow or pancreas are subjected to at least one denaturation operation for the extraction, wherein
- cells and/or tissues from cartilage, bone marrow or pancreas are provided,
- powdered, preferably in the deep-frozen state,
- defatted, preferably using an organic solvent,
- demineralized, preferably using acids,
- extracted using guanidine and/or guanidinium hydrochloride as chaotropic substances, and
- dialyzed, preferably against buffers, and
wherein differentiation is effected of
- mesenchymal stem cells to chondrocytes using cartilage extracts,
- mesenchymal stem cells to ß-cells using pancreas extracts, and/or
- hematopoietic stem cells to myeloid and erythroid cells using bone marrow extracts.

5. The use as claimed in claim 4, **characterized in that** the cartilage, bone marrow or pancreas are of human and/or animal origin.

6. The use as claimed in claim 4 or 5, **characterized in that** the active substances and/or components comprise at least leukotrienes, cytotactin, tenascin, laminin, fibronectin, cytokines, osteogenic active substances, in particular BMP-1, BMP-2, IGF1, TGF-β1, FGF and PDGF, collagen, in particular of type I.

7. The use as claimed in any of claims 4 to 6, **characterized in that** the active substances and/or components are present in combination and/or in a complex.

8. Use of mesenchymal and/or hematopoietic stem cells in a method of tissue engineering, comprising treatment of stem cells using extracts, comprising active substances and/or components which activate or inhibit growth and/or differentiation of stem cells to at least one tissue type, wherein the extracts may be prepared according to a method, wherein cartilage, bone marrow or pancreas are subjected to at least one denaturation operation for the extraction,
**characterized in that**
- cells and/or tissues from cartilage, bone marrow or pancreas are provided,
- powdered, preferably in the deep-frozen state,
- defatted, preferably using an organic solvent,
- demineralized, preferably using acids,
- extracted using guanidine and/or guanidinium hydrochloride as chaotropic substances,
and
- dialyzed, preferably against buffers, and
wherein differentiation is effected of
- mesenchymal stem cells to chondrocytes using cartilage extracts,
- mesenchymal stem cells to ß-cells using pancreas extracts, and/or
- hematopoietic stem cells to myeloid and erythroid cells using bone marrow extracts.

9. The use as claimed in claim 8, **characterized in that** the cartilage, bone marrow or pancreas are of human and/or animal origin.

10. The use as claimed in claim 8 or 9, **characterized in that** the active substances and/or components comprise at least leukotrienes, cytotactin, tenascin, laminin, fibronectin, cytokines, osteogenic active substances, in particular BMP-1, BMP-2, IGF1, TGF-β1, FGF and PDGF, collagen, in particular of type I.

11. The use as claimed in any of claims 8 to 10, **characterized in that** the active substances and/or components are present in combination and/or in a complex.

## Revendications

1. Procédé pour la différenciation au moins partielle de cellules souches mésenchymateuses et/ou hématopoïétiques en au moins un type de tissu, comprenant :
- le traitement des cellules souches avec des extraits qui présentent des substances actives et/ou des composants pour la différenciation de cellules souches, et
- la culture et la différenciation des cellules souches,
**caractérisé en ce que** pour la préparation des extraits, on soumet le cartilage, la moelle osseuse ou le pancréas à au moins un processus de dénaturation en vue de l'extraction, où
- on met à disposition des cellules et/ou un tissu provenant du cartilage, de la moelle osseuse ou du pancréas,
- on pulvérise, de préférence à l'état congelé,
- on dégraisse, de préférence à l'aide d'un solvant organique,
- on déminéralise, de préférence à l'aide d'acides,
- on extrait à la guanidine et/ou au chlorhydrate de guanidinium en tant que substances chaotropes et
- on dialyse, de préférence contre des tampons,
et où
- des cellules souches mésenchymateuses sont différenciées, en utilisant les extraits de cartilage, en chondrocytes,
- des cellules souches mésenchymateuses sont différenciées, en utilisant les extraits de pancréas, en cellules ß et/ou
- les cellules souches hématopoïétiques sont différenciées, en utilisant les extraits de moelle osseuse, en cellules myéloïdes et érythroïdes.

2. Procédé selon la revendication 1, **caractérisé en ce que** les cellules souches présentent, après le traitement par les extraits, en leur surface cellulaire, des molécules spécifiques pour des cellules de tissus déterminés et/ou une morphologie caractéristique pour des tissus déterminés.

3. Procédé selon la revendication 1 ou 2, **caractérisé en ce qu'**on utilise, pour le traitement, des extraits qui sont d'origine humaine et/ou animale.

4. Utilisation d'extraits, comprenant des substances actives et/ou des composants, qui activent ou inhibent la croissance et/ou la différenciation de cellules souches en au moins un type de tissu, pour la différenciation de cellules souches mésenchymateuses et/ou hématopoïétiques, pouvant être préparés selon un procédé, dans lequel on soumet le cartilage, la moelle osseuse ou le pancréas à au moins un processus de dénaturation en vue d'une extraction, où
- on met à disposition des cellules et/ou un tissu provenant du cartilage, de la moelle osseuse ou du pancréas,
- on pulvérise, de préférence à l'état congelé,
- on dégraisse, de préférence à l'aide d'un solvant organique,
- on déminéralise, de préférence à l'aide d'acides,
- on extrait à la guanidine et/ou au chlorhydrate de guanidinium en tant que substances chaotropes et
- on dialyse, de préférence contre des tampons,
et où
- des cellules souches mésenchymateuses sont différenciées, en utilisant les extraits de cartilage, en chondrocytes,
- des cellules souches mésenchymateuses sont différenciées, en utilisant les extraits de pancréas, en cellules ß et/ou
- les cellules souches hématopoïétiques sont différenciées, en utilisant les extraits de moelle osseuse, en cellules myéloïdes et érythroïdes.

5. Utilisation selon la revendication 4, **caractérisée en ce que** le cartilage, la moelle osseuse ou le pancréas est d'origine humaine et/ou animale.

6. Utilisation selon la revendication 4 ou 5, **caractérisée en ce que** les substances actives et/ou les composants comprennent au moins des leucotriènes, de la cytotactine, de la ténascine, de la laminine, de la fibronectine, des cytokines, des substances actives ostéogènes, en particulier BMP-1, BMP-2, IGF1, TGF-ß1, FGF et PDGF, du collagène, en particulier du type I.

7. Utilisation selon l'une quelconque des revendications 4 à 6, **caractérisée en ce que** les substances actives et/ou les composants se trouvent en combinaison et/ou dans un complexe.

8. Utilisation de cellules souches mésenchymateuses et/ou hématopoïétiques dans un procédé d'ingénierie tissulaire, comprenant un traitement de cellules souches avec des extraits, comprenant des substances actives et/ou des composants qui activent ou inhibent la croissance et/ou la différenciation de cellules souches en au moins un type de tissu, les extraits pouvant être préparés selon un procédé dans lequel on soumet le cartilage, la moelle osseuse ou le pancréas à au moins un processus de dénaturation en vue de l'extraction, **caractérisée en ce que**
- on met à disposition des cellules et/ou un tissu provenant du cartilage, de la moelle osseuse ou du pancréas,
- on pulvérise, de préférence à l'état congelé,
- on dégraisse, de préférence à l'aide d'un solvant organique,
- on déminéralise, de préférence à l'aide d'acides,
- on extrait à la guanidine et/ou au chlorhydrate de guanidinium en tant que substances chaotropes et
- on dialyse, de préférence contre des tampons,
et où
- des cellules souches mésenchymateuses sont différenciées, en utilisant les extraits de cartilage, en chondrocytes,
- des cellules souches mésenchymateuses sont différenciées, en utilisant les extraits de pancréas, en cellules ß et/ou
- les cellules souches hématopoïétiques sont différenciées, en utilisant les extraits de moelle osseuse, en cellules myéloïdes et érythroïdes.

9. Utilisation selon la revendication 8, **caractérisée en ce que** le cartilage, la moelle osseuse ou le pancréas est d'origine humaine et/ou animale.

10. Utilisation selon la revendication 8 ou 9, **caractérisée en ce que** les substances actives et/ou les composants comprennent au moins des leucotriènes, de la cytotactine, de la ténascine, de la laminine, de la fibronectine, des cytokines, des substances actives ostéogènes, en particulier BMP-1, BMP-2, IGF1, TGF-ß1, FGF et PDGF, du collagène, en particulier du type I.

11. Utilisation selon l'une quelconque des revendications 8 à 10, **caractérisée en ce que** les substances actives et/ou les composants se trouvent en combinaison et/ou dans un complexe.
